Europäisches Patentamt

European Patent Office

Office européen des brevets

(11) Publication number: **0 488 181 A1**

# EUROPEAN PATENT APPLICATION

(21) Application number: **91120201.8**

(22) Date of filing: **26.11.91**

(51) Int. Cl.5: **A61K 31/44**, A61K 9/48, A61K 9/66, A61K 9/14

(30) Priority: **29.11.90 JP 325443/90**

(43) Date of publication of application:
**03.06.92 Bulletin 92/23**

(84) Designated Contracting States:
**BE CH DE DK ES FR GB IT LI NL SE**

(71) Applicant: **Nippon Kayaku Kabushiki Kaisha**
**11-2, Fujimi-cho 1 chome Chiyoda-ku**
**Tokyo(JP)**

(72) Inventor: **Aoki, Minoru**
**29-16, Shimo-3-chome**
**Kita-ku, Tokyo(JP)**
Inventor: **Nakada, Minoru**
**90-1-207, Azuma-5-chome**
**Kitamoto-si(JP)**
Inventor: **Yazawa, Yuichi**
**4-9, Hirai-1-chome**
**Edogawa-ku, Tokyo(JP)**
Inventor: **Izu, Genichi**
**1213-69, Shimonoda, Shiraokamachi**
**Minamisaitama-gun, Saitama-ken(JP)**
Inventor: **Terada, Takashi**
**16-10, Hibarino-1-come**
**Konosu-shi(JP)**

(74) Representative: **Türk, Dietmar, Dr. rer. nat. et al**
**Türk, Gille + Hrabal Patentanwälte**
**Brucknerstrasse 20**
**W-4000 Düsseldorf 13(DE)**

(54) **Hard capsule preparation containing nifedipine.**

(57) This invention relates to a hard capsule preparation prepared by filling a hard capsule with a liquid composition comprising 1 part by weight of nifedipin, 5 to 25 parts by weight of a polyethylene glycol liquid at ambient temperature and 0.4 to 10 parts by weight of polyvinylpyrrolidone. Nifedipin is extensively in use as a therapeutic drug for angina pectoris.

EP 0 488 181 A1

F I G. I

FIELD OF THE INVENTION

This invention relates to a hard capsule preparation of nifedipin [1,4-dihydro-2,4-dimethyl-4-(2'-nitrophenyl)-3,5-dicarbomethoxypyridine] useful as a therapeutic drug for angina pectoria.

BACKGROUND OF THE INVENTION

Since nifedipin is only slightly soluble in water, it is poor in absorbability when administered orally in a crystal state or in the form of fine powder. Thus, a variety of studies have been conducted with the aim of improving its absorption. As a method for improving its absorption, there is known a method which comprises dissolving nifedipin into polyalkylene glycol or the like and making the solution into an soft capsule preparation (US Patent 3,784,684).

Its soft capsule preparation is disadvantageous in that the nifedipin contained in the inner fluid is deposited as a crystal upon elution into water. In order to prevent the deposition of nifedipin crystal, there is an attempt to add polyvinylpyrrolidone to the inner fluid (Japanese Patent Application KOKAI No. 60-105611).

However, soft capsule preparation has a fault that it tends to soften under some conditions of preservation and the capsule itself is readily deformable and adherent. Further, it has a problem that water migrates from the water-rich capsule skin to the inner fluid and results in deposition of nifedipin crystal, and for its prevention the dissolution medium must be used in a large quantity which results in increasing the capsule size and making the capsule difficult to take. On the other hand, as a method for facilitating absorption of nifedipin by forming it into a powder, there have been disclosed powdery or granular preparations of an amorphous solid solution prepared from nifedipin and polyvinylpyrrolidone (PVP) (EP-A-0001247 and US Patent 4,412,986). However, this type of preparations are said to have a problem of crystal transition caused by moisture absorption (IYAKUHIN KENKYU, 12 (4), 1981) and have a problem of residual solvent because they are prepared by the use of an organic solvent.

Apart from the above, there is known a hard capsule preparation of nifedipin prepared by filling a hard capsule with a composition comprising a solution-form polyethylene glycol and a non-solution-form polyethylene glycol (US Patent 4,894,235). In this type of capsule preparation, the composition filled into capsule solidifies in the capsule, so that it has a problem that the elution of nifedipin into water is lower than that in soft capsule and its in vivo absorbability is insufficient. As have been mentioned above, in the prior capsule preparations, those of which content is a liquid substance are soft capsules and those of which content is not liquid at ambient temperature are hard capsules. There is known no hard capsule filled with a substance which is liquid at ambient temperature.

BRIEF DESCRIPTION OF THE DRAWING

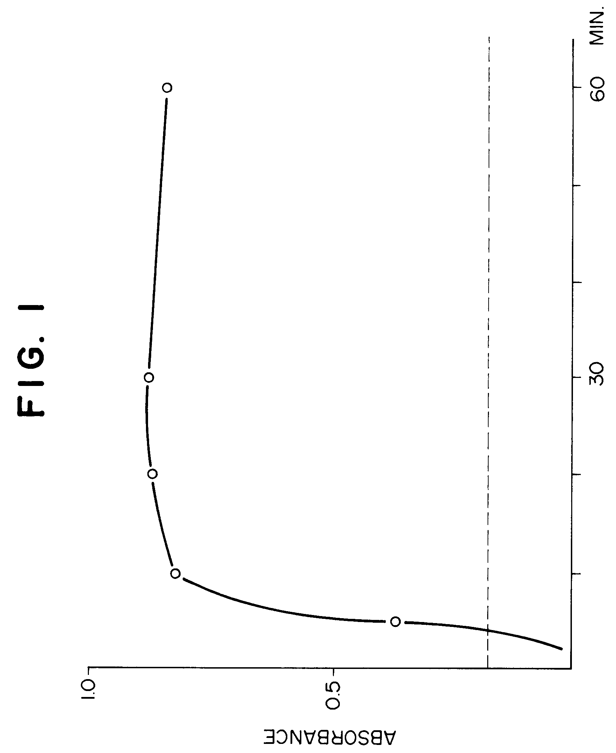

Figure 1 illustrates the relation between passage of time and absorbance in a case that the capsule of this invention is added to water, wherein "-----" signifies absorbance of a saturated aqueous solution of nifedipin.

SUMMARY OF THE INVENTION

This invention relates to a hard capsule preparation filled with a liquid composition comprising 1 part by weight of nifedipin (hereinafter referred to as NP), 5 to 25 parts by weight of polyethylene glycol which is liquid at ambient temperature and 0.4 to 10 parts by weight of polyvinylpyrrolidone (hereinafter referred to as PVP).

DETAILED DESCRIPTION OF THE INVENTION

Next, this invention will be explained in more detail. The solvents which can be used in this invention for the purpose of dissolving NP include polyethylene glycols which are liquid at ambient temperature, such as Polyethylene Glycol 300, Polyethylene Glycol 400, Polyethylene Glycol 600 and the like, among which Polyethylene Glycol 600 is particularly preferable. Polyethylene glycols can be used either singly or in combination of two or more members, without any trouble. The polyethylene glycol is used usually in an amount of 5 to 25 parts by weight and preferably in an amount of 7 to 18 parts by weight, per 1 part by weight of NP.

The PVP used in this invention is not particularly limited in molecular weight. However, those having a molecular weight of 10,000 to 1,000,000 are preferable, and those having a molecular weight of 20,000 to 100,000 are more preferable, and those having a molecular weight of about 20,000 to about 50,000 are most preferable. The amount of PVP is about 0.4 to about 10 parts by weight, preferably about 1 to about 8 parts by weight, and more preferably about 1.5 to about 6 parts by weight, all per one part by weight of NP.

In the inner solution to be filled into the hard capsule of this invention, proportions of ingredients are as follows. Thus, NP is used in an amount of 4 to 20% w/w and preferably 4.5 to 10% w/w; PVP is used in an amount of 5 to 45% w/w and preferably 8 to 30% w/w; and the polyethylene glycol is used in an amount of 50 to 90% w/w and preferably about 62 to about 82% w/w.

The inner solution has a viscosity of 1,000 to 100,000 cps, preferably 2,000 to 40,000 cps, and more preferably about 2,500 to about 25,000 cps at 25°C. For the purpose of increasing viscosity, a thickening agent such as hydroxypropylmethylcellulose acetate succinate and the like may be added in an amount of about 0.3 to about 0.8 part per one part of NP.

In producing the capsule preparation of this invention, PVP is dissolved into polyethylene glycol at a temperature of 20 to 100°C, preferably 40 to 80°C and thereafter NP is added thereto to prepare a liquid preparation. Then, the liquid is filled into a hard gelatin capsule at a temperature of 0 to 80°C, preferably at 20°C to 65°C, to prepare a capsule preparation. Then, if desired, a corrigent, an aromatizer and the like may be added to the solution each in an amount of about 1 to about 5% based on NP. The hard capsule used for this purpose may be usual white-colored opaque one or colored one.

It is entirely unnecessary to add water nor solvent other than polyethylene glycol to the inner solution of the capsule preparation of this invention.

Example 1

A sample solution of this invention having a viscosity of about 11,000 cps at 25°C was prepared by dissolving 1 g of NP and 4 g of PVP into 15 g of polyethylene Glycol 600 at a temperature of 70°C. Then, 200 mg of the sample solution thus obtained was filled into a No. 4 hard capsule at a temperature of 60°C and the capsule was band-sealed to obtain a hard capsule preparation of this invention.

Example 2

A sample solution having a viscosity of about 4,000 cps at 25°C was prepared by dissolving 10 g of NP and 25 g of PVP into 115 g of Polyethylene Glycol 600 at a temperature of 70°C. Then, 150 mg of the sample solution thus obtained was filled into a No. 4 hard capsule at a temperature of 50°C and the capsule was band-sealed to obtain a hard capsule preparation of this invention.

On 30 pieces of the hard capsule preparation thus obtained, weight of each capsule preparation was measured. As the result, mean value of the weight was 202.40 mg and the standard deviation was 0.85 mg.

Example 3

A sample solution having a viscosity of about 3,000 cps at 25°C was prepared by dissolving 10 g of NP and 20 g of PVP into 100 g of Polyethylene Glycol 400 at a temperature of 50°C. Then, 130 mg of the sample solution thus obtained was filled into a No. 5 hard capsule at a temperature of 40°C and the capsule was band-sealed to obtain a hard capsule preparation of this invention.

Example 4

A sample solution of this invention was prepared by dissolving 10 g of NP, 5 g of hydroxypropylmethylcellulose acetate succinate and 25 g of PVP K-25 into 150 g of Polyethylene Glycol 600 at a temperature of 80°C. Then, 200 mg of the sample solution thus obtained was filled into a No. 4 hard capsule at a temperature of 65°C and the capsule was band-sealed to obtain a hard capsule preparation of this invention.

Experiment Example

On the sample of Example 1, an elution test was carried out according to the Pharmacopoeia of Japan, 11th eddition (paddle method, rotation number 100, 37°C, distilled water). As shown in Figure 1, a good elution property was exhibited.

It is apparent from the description presented above that the hard capsule preparation of this invention contains NP at a higher concentration as compared with prior soft capsule preparations, and therefore its volume can be decreased, and it can be produced by a more easy and simple procedure less expensively. Further, even if it is thrown into water, no deposition of crystal is noticeable in spite of the high NP concentration, and no particular trouble is caused by the high NP concentration. Further, according to this invention, the accuracy of package into hard capsule is high, so that a capsule preparation almost free from unevenness of weight can be obtained.

Further, the hard capsule preparation of this invention can easily be produced without exudation of inner liquid composition out of the capsule.

Further, in the hard capsule preparation of this invention, the elution property of NP into water is good, so that a good in vivo absorbability, at least comparable to that of prior soft capsule preparations, can be expected.

**Claims**

1.  A hard capsule preparation prepared by filling a hard capsule with a liquid composition comprising 1 part by weight of nifedipin, 5 to 25 parts by weight of a polyethylene glycol liquid at ambient temperature and 0.4 to 10 parts by weight of polyvinyl pyrrolidone.

2. A hard capsule preparation according to Claim 1, wherein said liquid composition has a viscosity of 1,000 to 100,000 cps at 25°C.

3. A hard capsule preparation according to Claim 1, wherein said polyethylene glycol is Polyethylene Glycol 300, Polyethylene Glycol 400 or Polyethylene Glycol 600.

4. A hard capsule preparation according to Claim 1, wherein said polyvinylpyrrolidone has a molecular weight of 20,000 to 50,000.

5. A hard capsule preparation according to Claim 1, wherein the quantity of said polyethylene glycol is 7 to 18 parts by weigh and the quantity of said polyvinylpyrrolidone is 1.5 to 6 parts by weight, both per one part by weight of nifedipin.

6. A hard capsule preparation according to Claim 1, wherein proportions of the ingredients in the liquid composition are as follows: nifedipin 4.5 to 10% w/w, polyethylene glycol 62 to 82% w/w, polyvinylpyrrolidone 8 to 30% w/w.

**Claims for the following Contracting State :ES**

1. A process for producing a hard capsule preparation which comprises filling a hard capsule with a liquid composition comprising 1 part by weight of nifedipin, 5 to 25 parts by weight of a polyethylene glycol liquid at ambient temperature and 0.4 to 10 parts by weight of polyvinylpyrrolidone.

2. A Process according to Claim 1, wherein said liquid composition has a viscosity of 1,000 to 100,000 cps at 25°C.

3. A process according to Claim 1, wherein said polyethylene glycol is Polyethylene Glycol 300, Polyethylene Glycol 400 or Polyethylene Glycol 600.

4. A process according to Claim 1, wherein said polyvinylpyrrolidone has a molecular weight of 20,000 to 50,000.

5. A process according to Claim 1, wherein the quantity of said polyethylene glycol is 7 to 18 parts by weight and the quantity of said polyvinylpyrrolidone is 1.5 to 6 parts by weight, both per one part by weight of nifedipin.

6. A process according to Claim 1, wherein the proportions of ingredients in the liquid composition are as follows: nifedipin 4.5 to 10% w/w, polyethylene glycol 62 to 82% w/w, polyvinylpyrrolidone 8 to 30% by weight.

7. A process according to Claim 1, wherein the temperature at the time of filling is 0°C to 80°C.

FIG. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | EP-A-0 182 007 (DR. RENTSCHLER ARZNEIMITTEL GMBH ) | 1,2 | A61K31/44 A61K9/48 |
| Y,D | * page 3, paragraph 3 * <br> * page 4, paragraph 2 * <br> * page 4, paragraph 4 - page 5, paragraph 1 * <br> * page 9 - page 10; example 6 * <br> * claims * | 1-6 | A61K9/66 A61K9/14 |
| D,Y | DE-A-2 822 882 (YAMANOUCHI PHARMACEUTICAL CO LTD ) <br> * page 16; example 17 * | 1-5 | |
| Y | US-A-4 689 233 (STEPHAN DVORSKY ET AL) <br> * column 3; example 3 * <br> * claims 1,2,6 * | 4-6 | |
| Y | EP-A-0 278 168 (HARRIS PHARMACEUTICALS LTD) <br> * column 2; example 1 * <br> * claims 1,5 * | 3 | |
| X | EP-A-0 265 848 (DR. RENTSCHLER ARZNEIMITTEL GMBH ) <br> * page 3, line 46 - line 52 * <br> * page 4, line 1 - line 17 * <br> * page 4; example 1 * | 1 | TECHNICAL FIELDS SEARCHED (Int. Cl.5) <br><br> A61K |
| A,D | PATENT ABSTRACTS OF JAPAN <br> vol. 9, no. 247 (C-307)(1970) 3 October 1985 <br> & JP-A-60 105 611 ( NIPPON CHEMIPHER KK ) 11 June 1985 <br> * abstract * | 1-6 | |
| A | PATENT ABSTRACTS OF JAPAN <br> vol. 7, no. 11 (C-145)(1156) 18 January 1983 <br> & JP-A-57 167 911 ( KIYOUTO YAKUHIN KOGYO KK ) 16 October 1982 <br> * abstract * | 1-6 | |

-/--

The present search report has been drawn up for all claims

| Place of search | Date of completion of the search | Examiner |
|---|---|---|
| THE HAGUE | 07 FEBRUARY 1992 | BOULOIS D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent
Office

EUROPEAN SEARCH REPORT

Application Number

EP    91 12 0201
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 802 625 (R.P. SCHERER CORPORATION)<br>* page 10, line 1 - line 19 *<br>* page 14, line 27 - page 15, line 6 *<br>* page 16; table 4 * | 1-6 | |

-----

TECHNICAL FIELDS
SEARCHED (Int. Cl.5)

The present search report has been drawn up for all claims

| Place of search | Date of completion of the search | Examiner |
|---|---|---|
| THE HAGUE | 07 FEBRUARY 1992 | BOULOIS D. |

EPO FORM 1503 03.82 (P0401)